# EUROPÄISCHE PATENTSCHRIFT

(11) **EP 1 725 881 B1**
(45) Veröffentlichungstag und Bekanntmachung des Hinweises auf die Patenterteilung: **29.04.2009**
(21) Anmeldenummer: 05715805.7
(22) Anmeldetag: 08.03.2005
(51) Int. Cl.: G01N 33/487

(54) **TESTELEMENT-ANALYSESYSTEM MIT HARTSTOFFBESCHICHTETEN KONTAKTFLÄCHEN**
TEST ELEMENT ANALYSIS SYSTEM WITH CONTACT SURFACES COATED WITH HARD MATERIAL
SYSTEME D'ANALYSE A ELEMENTS D'ESSAI, POURVU DE SURFACES DE CONTACT A REVETEMENT EN MATIERE DURE

(30) Priorität: 10.03.2004 DE 102004011648
(43) Veröffentlichungstag der Anmeldung: 29.11.2006
(73) Patentinhaber: Roche Diagnostics GmbH, 68305 Mannheim (DE); F.HOFFMANN-LA ROCHE AG, 4070 Basel (CH)
(72) Erfinder: DREIBHOLZ, Joerg, 67122 Altrip (DE); RIEBEL, Stefan, 76756 Bellheim (DE); AUGSTEIN, Manfred, 68305 Mannheim (DE)
(86) Internationale Anmeldenummer: PCT/EP2005/002397
(87) Internationale Veröffentlichungsnummer: WO 2005/088319

(56) Entgegenhaltungen:
- US-A- 6 029 344
- US-A- 6 046 051
- PATENT ABSTRACTS OF JAPAN Bd. 015, Nr. 402 (P-1262), 14. Oktober 1991 (1991-10-14) & JP 03 162660 A (OMRON CORP), 12. Juli 1991 (1991-07-12)
- "Roempp Online" [Online] März 2002 (2002-03), GEORG THIEME VERLAG , STUTTGART , XP002328486 Gefunden im Internet: URL:www.roempp.com/prod/roempp.php> [gefunden am 2005-05-18] "Stichwort Hartstoffe"

## Beschreibung

### Gebiet der Erfindung

Die Erfindung betrifft ein Testelement-Analysesystem zur analytischen Untersuchung einer Probe, insbesondere einer Körperflüssigkeit von Menschen oder Tieren. Zu dem System gehören mindestens zwei Bestandteile, nämlich ein Testelement, welches eine Messzone aufweist, in welche die zu untersuchende Probe zur Durchführung einer Analyse gebracht wird, um eine für die Analyse charakteristische Messgröße zu messen, und ein Auswertegerät mit einer Testelementhalterung, um das Testelement in einer Messposition zur Durchführung der Messung zu positionieren und einer Messvorrichtung zur Messung der charakteristischen Messgröße.

### Stand der Technik

Testelement-Analysesysteme sind insbesondere in der medizinischen Diagnostik für die Analyse von Körperflüssigkeiten wie Blut oder Urin gebräuchlich. Die zu untersuchenden Probe wird zunächst auf ein Testelement aufgebracht. Dort finden die zum Nachweis des Analyten notwendigen Verfahrensschritte, meist chemische, biochemische, biologische oder immunologische Nachweisreaktionen oder physikalische Wechselwirkungen, statt, welche zu einer charakteristischen und messbaren Veränderung des Testelements insbesondere im Bereich der Messzone führen. Zur Bestimmung dieser charakteristischen Veränderung wird das Testelement in ein Auswertegerät eingebracht, welches die charakteristische Veränderung des Testelements bestimmt und in Form eines Messwertes zur Anzeige oder Weiterverarbeitung bereitstellt.

Testelemente sind häufig als Teststreifen ausgebildet, welche im wesentlichen aus einer länglichen Trägerschicht, meist aus einem Kunststoffmaterial, und einer Messzone mit einer die Nachweisreagenzien enthaltenen Nachweisschicht und evtl. weiteren Hilfsschichten, beispielsweise Filtrationsschichten, bestehen. Weiterhin enthalten die Testelemente der vorliegenden Erfindung Kontaktflächen, über welche ein elektrischer Kontakt zwischen dem Testelement und dem Auswertegerät hergestellt werden kann. Im Falle elektrochemischer Analyseverfahren sind insbesondere Leiterbahnen und Elektroden auf dem Testelement aufgebracht. Auch Testelemente, welche keine elektrochemischen Analysemethoden einsetzen, können elektrisch leitende Kontaktflächen besitzen, beispielsweise zur Übertragung von auf dem Testelement gespeicherten Kalibrationsdaten oder Chargeninformationen an das Auswertegerät.

Die zugehörigen Auswertegeräte besitzen Testelementhalterungen mit speziellen Kontaktelementen, welchen einen elektrisch leitenden Kontakt zwischen dem Testelement und der Mess- und Auswerteelektronik des Auswertegerätes herstellen. Diese Kontaktelemente sind meist als elektrische Steckverbindungen mit metallischen Federelementen ausgebildet, welche oft mit einer Edelmetalloberfläche, meist aus Gold oder Platin, versehen sind. Die Teststreifen werden zur Messung in die Testelementhalterung geschoben, wobei die Kontaktflächen der Kontaktelemente über die Elektroden der Testelemente hinwegbewegt werden. In einer Endposition befindet sich dann die Kontaktfläche der Kontaktelemente des Auswertegeräts in Kontakt mit der Kontaktfläche des Testelements. Durch eine insbesondere durch die Form und Federkraft des Kontaktelements definierte Andruckkraft kommt es zu einer elektrisch leitenden Verbindung zwischen Testelement und Auswertegerät. Hierbei soll insbesondere ein möglichst geringer und konstanter Übergangswiderstand zwischen der Kontaktfläche des Kontaktelements des Auswertegeräts und der Kontaktfläche des Testelements gewährleistet sein, um eine exakte und reproduzierbare Signalübertragung zu ermöglichen. Ein konstanter und reproduzierbarer Übergangswiderstand ist insbesondere wichtig, um auch nach vielen zuvor erfolgten Steckungen eines Testelements noch exakte Messresultate zu erzielen und so eine hohe und reproduzierbare Messgenauigkeit zu erzielen, insbesondere in Betracht dessen, dass solche Testelement-Analysesysteme oft über viele Jahre eingesetzt werden oder viele zehntausend Steckungen erfolgen. Dies ist insbesondere in klinischen Bereich von großer Bedeutung, wo derartige Testsysteme oft einen hohen Durchsatz bewältigen müssen.

Ein großer Vorteil steckbarer Kontaktvorrichtungen besteht in der leichten Zusammenfüg- und Trennbarkeit der elektrischen Verbindung, so dass Testelement und Auswertegerät unabhängig voneinander aufbewahrt und eingesetzt werden können. Da die Kontaktflächen einerseits einen möglichst optimalen Übergang des elektrischen Stroms gewährleisten sollen, was einen gewissen Kontaktdruck erfordert, andererseits durch das Zusammenfügen der Kontaktverbindung, insbesondere aber durch ein wiederholtes Zusammenfügen und Trennen der Kontaktverbindung, stark beansprucht werden, werden die Kontaktflächen häufig mit einer Edelmetallschicht, beispielsweise durch Plattierung oder Galvanisierung mit Gold, Silber, Platin oder Palladium, versehen Die oft hohe mechanische Belastung der Kontaktflächen, insbesondere durch Abrieb, Ablagerungen oder das Verkratzen der Kontaktflächen stellt auch deshalb ein Problem dar, weil ein gewisser Kontaktdruck für einen sicheren elektrischen Kontakt gewährleistet sein muss und ein gewisser Steckweg des Testelements aus mechanischen Gründen, insbesondere einer Sicherheit der Führung beim Stecken und einer mechanische Stabilität im gesteckten Zustand, nötig ist. Für eine hohe Kontaktsicherheit zwischen den Kontaktflächen einer elektrischen Kontaktverbindung und im Hinblick auf möglichst geringe Kontaktwiderstände ist es von großer Bedeutung, dass die Kontaktflächen gegen äußere Einflüsse möglichst widerstandsfähig sind. Die äußeren Einflüsse können dabei chemischer, physikalischer oder mechanischer Natur sein. So kommt es insbesondere beim Steckvorgang zum Aneinänderreiben der beiden Kontaktflächen, wodurch diese mechanisch sehr stark beansprucht werden. Auch wirken Korrosionseffekte, insbesondere Spaltkorrosion, negativ auf die Kontaktsicherheit und den Kontaktwiderstand ein. Ein weiteres Problem solcher Testelement-Analysegeräte ist es, dass das Trägermaterial der eingesetzten Testelemente oft aus einer elastischen und relativ weichen Kunststofffolie besteht, auf welche die Kontaktflächen und Elektroden aufgebracht werden, so dass sich durch diesen Aufbau auf einem relativ weichem Untergrundmaterial Nachteile für eine exakte Kontaktierung ergeben können.

Ein großer Nachteil von Edelmetall-Edelmetall-Paarungen bei Kontaktflächen solcher Steckverbindungen besteht darin, dass es, auch unabhängig von deren Geometrie und/oder der Andruckkraft, sehr häufig bei zu einer Beschädigung der Metalloberflächen beim Zusammenfügen der Kontaktflächen und damit zu elektrischen Kontaktproblemen kommt. Solche Kontaktprobleme äußern sich oft darin, dass die Übergangswiderstände zwischen Stecker und Kontaktelement hochohmig werden oder im Extremfall gar kein elektrischer Kontakt mehr zwischen den Bestandteilen der Kontaktverbindung existiert. Mikroskopisch betrachtet zeigt sich vor allem bei flächigen Kontakten wie Leiterbahnen oder Elektroden häufig ein Schädigungsbild, welches nach der Steckung durch eine starke Veränderung der Dicke der Metallschicht dieser Kontaktfläche gekennzeichnet ist. So wird in manchen Bereichen die Metallschicht der Elektroden durch die sich darüber hinwegbewegende zweite Kontaktfläche stark verformt, insbesondere in Form von Rillen, Riefen und Kratzern. Dieses Schädigungsbild tritt dann vor allem auf, wenn die Elektroden auf relativ weichen Grundmaterialien aufgebracht sind. Diese Verformungen können so groß werden, dass in manchen Bereichen die Metallschicht durch das Darüberhinwegbewegen der zweiten Kontaktfläche ganz abgetragen wird. In diesem Fall ist kein elektrischer Kontakt zwischen Testelement und Auswertegerät mehr möglich. Derartige Verformungen von Metallschichten, welche als Kontaktflächen dienen, äußern sich in nicht definierten und deutlich erhöhten Übergangswiderständen oder dem gänzlichen Fehlen eines elektrischen Kontakts. Solche Kontaktelemente sind daher für den Einsatz in Analysesystemen, welche eine reproduzierbare Bestimmung eines Analyten über einen lange Einsatzdauer hinweg gewährleisten sollen, ungeeignet.

Zur Überwindung dieser Nachteile wurden deshalb im Stand der Technik folgende Lösungsansätze gegeben:

Um eine hohe Kontaktsicherheit von Steckverbindungen insbesondere bei hohen mechanischen und/oder chemischen Belastungen zu gewährleisten, beschreibt DE 102 22 271 A1 ein Verfahren zur Erhöhung der mechanischen und/oder chemischen Widerstandsfähigkeit einer elektrischen Kontaktverbindung zwischen zwei Kontaktteilen, bei dem zumindest eines der Kontaktteile mit Hilfe eines thermischen Spritzverfahrens im Bereich der Kontaktflächen beschichtet wird. Ziel dieser Anmeldung ist es, durch diese Beschichtung den Abrieb der Kontaktfläche zu minimieren. Als Anwendungsgebiet solcher Kontaktverbindungen sind dort Steckverbindungen elektronischer Bauteile, wie Leiterplatten und Platinen, oder Schleifkontakte, beispielsweise in Motoren, gebannt. Solche Kontaktverbindungen sind vor allem dadurch gekennzeichnet, dass die Kontaktverbindung nach einmalig erfolgter Kontaktierung der beteiligten Kontaktflächen andauernden hohen mechanischen Belastungen ausgesetzt sind, beispielsweise durch Vibrationen oder andauerndes Aneinanderschleifen der Kontaktflächen, so dass es dort zu starkem Abrieb der beteiligten Kontaktflächen kommt. Ziel dieser Anmeldung ist es insbesondere, den Abrieb der Kontaktflächen selbst zu minimieren und weniger einen zuverlässigen elektrischen Kontakt der Kontaktflächen auch nach vielfachen Zusammenfügen und Trennen der Kontaktverbindung zu gewährleisten. Als Beschichtungsstoffe sind dort widerstandsfähige Metalllegierungen wie Bronze genannt, welche auf eine oder beide Kontaktflächen aufgebracht werden, um so den Abrieb dieser Kontaktflächen selbst zu vermindern. Die Beschichtung selbst erfolgt mit thermischen Spritzverfahren. Solche Verfahren, welche mit hohen Temperaturen arbeiten, sind für Testelemente, deren Testträger sehr häufig aus dünnen Kunststofffolien bestehen, ungeeignet, da derartige Kunststofffolien nicht die benötigte Hitzestabilität besitzen. Die Schichtdicken der Beschichtungsschicht müssen mit 10 µm bis 200 µm relativ stark sein, um eine dauerhafte Verbindung auch bei hoher Belastung und weiterhin unvermeidlichen Abrieberscheinungen zu ermöglichen. Zu solch erhöhten Abrieberscheinungen kommt es insbesondere, wenn beide Kontaktflächen mit einer solchen widerstandsfähigen Beschichtung versehen sind.

Die europäische Patentanmeldung EP 0 082 070 beschreibt ebenfalls ein Verfahren zum Schutz elektrischer Kontaktverbindungen, insbesondere von Schaltern und Relais. Ziel dieser Anmeldung ist es, Metalle, speziell Metallkontakte, durch eine Beschichtung vor Verschleiß zu schützen. Die Beschichtung soll analog zu DE 102 22 271 A1 dazu dienen, die Kontaktflächen gegen Abrieb widerstandsfähig zu machen. Hierzu wird auf die bestehenden Metallkontakte eine Schicht von Titannitrid aufgebracht, welche durch folgende Merkmale gekennzeichnet ist: eine Adhäsion von mehr als 180 kg/cm², hohe chemische Beständigkeit, hohe Abriebfestigkeit und einen spezifischen Widerstand von ca. 500 µΩ*cm. Auch hier dient die Beschichtung dazu, den Abrieb der Kontaktflächen selbst zu minimieren und weniger dazu, einen zuverlässigen elektrischen Kontakt der Kontaktflächen auch nach vielfachen Zusammenfügen und Trennen der Kontaktverbindung zu gewährleisten.

US 6,029,344 beschreibt federnde Kontaktelemente, besonders zur elektrischen Kontaktierung von elektronischen Bauteilen, welche mit einem "Hartstoff" überzogen sind. Ziel ist es hierbei, die mechanischen Eigenschaften der Kontaktverbindung durch den "Hartstoff"-Überzug zu modifizieren. Insbesondere sollen hierdurch die elastischen Eigenschaften des Kontaktelements verbessert werden. Die Beschichtung dient hier nicht primär einer Verminderung des Abriebs der Kontaktflächen oder einer besseren Kontaktsicherheit, sondern der Modifizierung der elastischen Eigenschaften der Federkontakte. Hierzu werden die aus relativ weichen Grundmaterialien, wie beispielsweise Gold, bestehenden Federkontakte zumindest in den Bereichen, welche derartig geformt sind, dass sie eine Federwirkung des Kontaktelements ermöglichen, mit einem Stoff beschichtet sind, welcher eine höhere Streckfestigkeit als das Grundmaterial aufweist. Als solche Stoffe werden insbesondere Metalle wie Nickel, Kupfer, Kobalt, Eisen, Gold, Silber, Elemente der Platingruppe und sonstige Edelmetalle, Halbedelmetalle, Wolfram, Molybdän, Zinn, Blei, Bismut und Indium sowie deren Legierungen genannt. Diese Stoffe werden im Sinne von US 6,029,344 als "Hartstoffe" (hard materials) bezeichnet und als Stoffe definiert, welche eine Streckfestigkeit vom mehr als 80.000 psi besitzen. Im Sinne der vorliegenden Anmeldung sind Hartstoffe gänzlich anders definiert. Solche "Hartstoffe" nach US 6,029,344 sind nicht dazu geeignet, die Anforderungen an eine sehr hohe Abriebsfestigkeit und hohe Kontaktsicherheit auch bei vielfachen Steckungen zu gewährleisten, sondern dienen der Verbesserung der elastischen Eigenschaften des Federkontakts. Die Schichtdicken der "Hartstoff"-Beschichtung müssen nach US 6,029,344 zwischen ca. 6 und 125 µm und mindestens ein Fünftel bis dem Fünffachen der Schichtdicke des Grundmaterials der Federkontakte betragen, um die Verbesserung der mechanischen, insbesondere der elastischen Eigenschaften des Kontaktelements zu bewirken.

Hartstoffbeschichtete Kontaktelement eines Sensors sind aus JP03162660 bekannt.

Die vorangehend beschrieben Dokumente beschreiben Verfahren zur Beschichtung von Oberflächen von elektrischen Kontaktelementen, welche entweder der Verringerung des Abriebs der Kontaktflächen selbst oder der Verbesserung der elastischen Eigenschaften des Kontaktelements dienen. Ein grundlegendes Problem, welches mit den vorgenannten Verfahren und Vorrichtungen nicht zufrieden stellend gelöst werden kann, besteht in der Gewährleistung einer zuverlässigen und über einen langen Zeitraum definierten elektrischen Verbindung zwischen den Kontaktflächen eines Kontaktelements, insbesondere bei hohen mechanischen Belastungen und auch nach zahlreichen erfolgten Kontaktierungsvorgängen.

### Aufgabe der Erfindung

Eine Aufgabe der vorliegenden Erfindung ist es, die Nachteile des Standes der Technik zu beseitigen oder zumindest zu verringern. Insbesondere soll ein einfach zu handhabendes Testelement-Analysesystem zu Verfügung gestellt werden, welches eine möglichst fehlerfreie Bestimmung des Analyten auch nach vielen erfolgten Steckungen eines Testelements in das Auswertegerät gewährleistet. Insbesondere soll eine Kontaktverbindung eines Testelement-Analysesystems zu Verfügung gestellt werden, welche einen über viele tausend Steckungen hinweg definierten und reproduzierbaren Übergangswiderstand zwischen Testelement und Auswertegerät und somit eine exakte und reproduzierbare Signalübertragung und Analytbestimmung über die gesamte Lebensdauer eines solchen Systems gewährleistet

Dies wird durch den Gegenstand der Erfindung, wie er in den Patentansprüchen und der Beschreibung charakterisiert ist, erreicht.

### Erfindungsgemäße Lösung

Gegenstand der Erfindung ist ein Testelement-Analysesystem zur analytischen Untersuchung einer Probe, insbesondere einer Körperflüssigkeit, mindestens umfassend ein Testelement mit einer oder mehreren Messzonen und sich auf dem Testelement befindlichen Kontaktflächen, insbesondere Elektroden oder Leiterbahnen, wobei die zu untersuchende Probe zur Durchführung einer Analyse in die Messzone gebracht wird, um eine für die Analyse charakteristische Messgröße zu bestimmen, sowie ein Auswertegerät mit einer Testelementhalterung zur Positionierung des Testelements in einer Messposition und einer Messvorrichtung zur Messung der charakteristischen Veränderung, wobei die Testelementhalterung Kontaktelemente mit Kontaktflächen enthält, welche einen elektrischen Kontakt zwischen dem Kontaktflächen des Testelements und den Kontaktflächen der Testelementhalterung ermöglichen. Die erfindungsgemäße Lösung besteht in der Beschichtung einer Kontaktfläche der Kontaktverbindung eines Testelement-Analysegeräts mit einem elektrisch leitenden Hartstoff.

Die Hartstoffoberfläche eines an der Kontaktverbindung beteiligten Elements kann dadurch gegeben sein, dass das gesamte Element oder ein Teil dieses Elements aus einem Hartstoff besteht. Da reine Hartstoffelemente oft unvorteilhafte mechanische und chemische Eigenschaften wie Sprödigkeit, schlechte elastische Eigenschaften oder auch einen relativ hohen elektrischen Widerstand, speziell bei hohen Materialstärken des Hartstoffes, aufweisen, wird in einer bevorzugten Ausführungsform die Hartstoffoberfläche durch Beschichtung eines Grundmaterials mit einem elektrisch leitenden Hartstoff gebildet. In der vorliegenden Erfindung werden deshalb vorwiegend dünne Hartstoffschichten als Kontaktflächen beschrieben. Die in der vorliegenden Erfindung für diese Hartstoffschichten beschriebenen Eigenschaften und Einsatzbereiche können jedoch auch auf Oberflächen von Elementen übertragen werden, welche ganz oder in großen Teilen aus einem Hartstoff bestehen.

Überraschenderweise zeigt sich, dass durch die Beschichtung einer Kontaktfläche mit einem elektrisch leitenden Hartstoff ein definierter und reproduzierbarer elektrischer Kontakt zwischen Testelement und Auswertegerät, insbesondere auch nach vielen erfolgten Steckungen, gewährleistet ist. Überraschenderweise zeigt die Beschichtung einer Kontaktfläche mit einem elektrisch leitenden Hartstoff deutlich verbesserte Kontakteigenschaften gerade gegenüber den bisher verbreitet eingesetzten Kontaktverbindungen, welche häufig auf beiden Seiten Kontaktflächen aus Edelmetall besitzen oder bei welchen oft beide Kontaktflächen mit Materialien beschichtet sind, welche den Abrieb der Kontaktflächen vermindern sollen. Erstgenannte werden häufig in zum einmaligen Gebrauch bestimmten Testelementen eingesetzt, letztere vor allem bei Kontaktverbindungen, welche für andauernde Kontaktierungsvorgänge aufgelegt sind und/oder hohen mechanischen Belastungen unterliegen.

Im Gegensatz zu metallischen Kontaktflächen besitzen Kontaktflächen, welche mit einer Hartstoffoberfläche versehen sind, insbesondere folgende Vorteile: Sie besitzen keramische Eigenschaften wie eine sehr hohe Härte, sind sehr beständig gegenüber chemischen Einflüssen, zeigen sehr gute Gleiteigenschaften über Oberflächen und weisen nur äußerst geringe Verschleiß-, Auflagerungs- oder Abriebsraten auf. Ihre hohe Benetzbarkeit durch metallische Schmelzen gewährleistet einen sehr hohen Zusammenhalt zwischen der Hartstoffschicht und einer darunter liegenden Metallschicht und eignet sich so sehr gut für den Einsatz in Verbundssystemen. Weiterhin weisen metallische Hartstoffe gute elektrische Eigenschaften wie eine hohe elektrische Leitfähigkeit auf, so dass sie als Oberflächenmaterial für elektrische Kontaktverbindungen besonders in Testelement-Analysesystemen sehr gut geeignet sind.

Unter Hartstoffen versteht man im Sinne der vorliegenden Anmeldung Stoffe, die aufgrund ihres spezifischen Bindungscharakters eine hohe Härte, insbesondere eine Vickers-Härte >1000 kp/mm², aufweisen. Der Schmelzpunkt von Hartstoffen liegt zumeist über 2000°C, ihre chemische und mechanische Beständigkeit ist gut und der keramischer Werkstoffe vergleichbar. Insbesondere umfasst der Begriff Hartstoffe im Sinne der vorliegenden Anmeldung metallische Hartstoffe. Diese zeichnen sich durch metallische Eigenschaften wie Glanz und elektrische Leitfähigkeit aus. Zu den metallischen Hartstoffen zählen insbesondere Carbide, Boride, Nitride und Silicide hochschmelzender Metalle wie Chrom, Zirconium, Titan, Tantal, Wolfram oder Molybdän einschließlich ihrer Mischkristalle und Komplexverbindungen. Insbesondere sind auch Modifikationen der oben genannten Hartstoffe eingeschlossen, welche weitere Zusätze anderer metallischer oder nichtmetallischer Stoffe zur weiteren Optimierung ihrer physikalischen und chemischen Eigenschaften, oft in geringen Konzentrationen, enthalten. Solche komplexeren Hartstoffverbindungen können insbesondere Aluminiumnitride, Carbonitride oder Carbidkohlenstoffe der oben genannten Metalle sein. Diese Definition von Hartstoffen entspricht weitestgehend der Definition aus dem Römpp Lexikon Chemie (Thieme Verlag Stuttgart, 10. Auflage 1996). Der im Rahmen der vorliegenden Erfindung zur Beschichtung eingesetzte Hartstoff soll elektrisch leitende Eigenschaften besitzen, so dass ein geringer Übergangswiderstand zwischen den Kontaktflächen der Testelementhalterung des Auswertegeräts und des Testelements gewährleistet ist, welcher eine exakte und reproduzierbare Signalübertragung ermöglicht. Insbesondere soll der Übergangswiderstand zwischen den Kontaktflächen des Testelements und den Kontaktflächen der Testelementhalterung kleiner als 50 Ohm sein.

Überraschenderweise zeigt sich, dass insbesondere solche metallischen Hartstoffe als vorteilhafte erfindungsgemäße Oberflächenmaterialien eingesetzt werden können, da sie weitere für den Einsatz in Kontaktelementen von Testelement-Analysegeräten vorteilhafte Eigenschaften wie hohe mechanische Härte, hohe chemische Beständigkeit, sehr gute Gleiteigenschaften und einen geringen Verschleiß aufweisen.

Besonders bevorzugte Hartstoffoberflächenmaterialien von Kontaktflächen im Rahmen der vorliegenden Erfindung sind metallische Nitride, insbesondere Titannitrid, Titanaluminiumnitrid, Chromnitrid oder Zirconiumnitrid.

Erfindungsgemäß ist eine der Kontaktflächen des Kontaktelements der Testelementhalterung des Auswertegeräts mit einer elektrisch leitenden Hartstoffoberfläche versehen. Unter Kontaktflächen sind im Rahmen der vorliegenden Anmeldung die elektrisch leitenden Strukturen des Kontaktelements zu verstehen, welche zur Herstellung eines elektrischen Kontakts zwischen Testelement und Auswertegerät in direkten Kontakt gebracht werden. Die Kontaktflächen des Kontaktelements können besonders ausgeformt sein, beispielsweise als flächige Elemente zur Erzielung einer möglichst großen Kontaktfläche und damit einer hohen Kontaktsicherheit und eines geringen Übergangswiderstands. Auch sind gekrümmte Ausformungen dieser Kontaktflächen möglich, um beispielsweise bei Feder- oder Steckkontakten ein möglichst einfaches und wenig schädigendes Einbringen des Testelements zu ermöglichen.

In einer bevorzugten Ausführungsform eines erfindungsgemäßen Testelement-Analysesystems sind die Kontaktflächen der Kontaktelemente der Testelementhalterung mit einer elektrisch leitenden Hartstoffoberfläche versehen.

Die Kontaktelemente, welche Bestandteile der Testelementhalterung des Auswertegerätes sind, können in verschiedenster Weise geformt sein. Sie können beispielsweise als Schleifkontakte, Rollkontakte, Steckkontakte, Federkontakte, Klemmkontakte oder Nullkraft-Kontakte ausgebildet sein. Insbesondere bei Kontaktelementtypen, bei welchen die Kontaktflächen der beiden an der Kontaktverbindung beteiligten Elemente bis zur Erreichung ihrer endgültigen Stellung unter direktem Kontakt aneinander vorbeibewegt werden, beispielsweise bei Steck-, Feder- oder Klemmkontakten, kann sich die erfindungsgemäße Ausgestaltung der Kontaktflächen besonders vorteilhaft auf die Kontaktsicherheit auswirken. Besonders bevorzugte Ausführungsformen der Kontaktelemente sind Steckkontakte, Federkontakte und Klemmkontakte. Verschiedenste mögliche Ausführungsformen solcher Kontaktelemente sind in US 6,029,344 dargestellt.

Ist die Hartstoffoberfläche als eine Beschichtung ausgebildet, kann das sich unter der Hartstoffbeschichtung befindliche Grundmaterial der Kontaktelemente prinzipiell jedes elektrisch leitende Material sein. Besonders eignen sich hierfür Metalle und Metalllegierungen, welche neben einer hohen elektrischen Leitfähigkeit weiterhin eine hohe chemische und mechanische Widerstandsfähigkeit besitzen. Typischerweise werden als Grundmaterialen von Steckverbindungen Kupferlegierungen eingesetzt, beispielsweise CuZn- oder CuSn-Legierungen sowie niedriglegierte Kupferwerkstoffe wie beispielsweise CuAg, CuCrSiTi oder CuMg. Im Falle federnder Kontaktelemente sollten die Grundmaterialien auch elastische Eigenschaften aufweisen.

Eine Hartstoffbeschichtung kann prinzipiell mit verschiedensten dem Fachmann bekannten Beschichtungsverfahren auf das Grundmaterial aufgebracht werden. Solche Verfahren sind beispielsweise Verfahren, bei welchen Stoffe aus flüssigen Lösungen auf Oberflächen abgeschieden werden, elektrochemische Metallisierungs- oder Galvanisierungsverfahren, nicht elektrochemische Metallisierungsverfahren, chemische Abscheideverfahren wie Chemical Vapor Deposition (CVD), physikalische Abscheideverfahren wie Physical Vapor Deposition (PVD), insbesondere mittels Evaporationsverfahren, Sputtering-Verfahren oder Laserablationsverfahren, oder Verfahren, welche auf der Zersetzung fester, flüssiger oder gasförmiger Stoffe beruhen.. Besonders bevorzugt können PVD-Sputtering-Verfahren zur Hartstoffbeschichtung eingesetzt werden.

Beim Aufbringen einer Hartstoffschicht auf das Grundmaterial des Kontaktelements kann es vorteilhaft sein, zunächst eine oder mehrere Zwischenschichten, insbesondere Keim- oder Schutzschichten, auf das Grundmaterial und auf diese anschließend die Hartstoffschicht aufzubringen. Durch das Aufbringen solcher Zwischenschichten kann insbesondere eine gute Haftung oder eine dauerhafte Haltbarkeit der Verbindung zwischen den unterschiedlichen Materialien erzielt werden. So können beispielsweise zunächst mit galvanischen Verfahren Schichten auf das Grundmaterial aufgebracht werden, welche eine besonders geeignete Oberfläche für die nachfolgende Hartstoffbeschichtung erzeugen. Weiterhin können auch Schutzschichten aufgebracht werden, welche bei einer Verletzung der Hartstoffoberfläche das darunter liegende Grundmaterial von chemischen und/oder physikalischen Schädigungen, beispielsweise Korrosion, schützen können. Weiterhin können durch eine geeignete Materialwahl solcher Zwischenschichten die elektrischen Eigenschaften des Kontaktelements, beispielsweise der Übergangswiderstand, beeinflusst werden. Zur Erzeugung solcher Zwischenschichten können beispielsweise Partikel aus einem geeigneten Material aufgetragen werden. Zur Erzielung einer guten und dauerhaften Verbindung zwischen Grundmaterial und Hartstoffschicht kann alternativ auch ein zusätzlicher Zwischenschritt vorgesehen sein, bei welchem die Oberfläche des Grundmaterials des Kontaktelements vor dem Beschichten so behandelt wird, so dass sie verbesserte Beschichtungseigenschaften aufweist.

Die Dicke und Zusammensetzung der Hartstoffschicht kann durch geeignete Wahl der Beschichtungsverfahren und deren Verfahrensparameter wie Temperatur, Verdampfungsrate, Zusammensetzung des Sputtertargets oder Dauer des Beschichtungsprozesses beeinflusst werden. Überraschenderweise hat sich gezeigt, dass gerade sehr dünne Schichten aus metallischen Hartstoffen einerseits sehr gute mechanische Eigenschaften, insbesondere eine hohe Härte und gute Gleitfähigkeit, andererseits jedoch auch gute elektrische Eigenschaften, insbesondere einen geringen elektrischen Widerstand aufweisen. Die bisher zur Beschichtung von Flächen eingesetzten Hartstoffe werden zumeist in wesentlich größeren Schichtdicken auf das Grundmaterial aufgebracht. So beschreibt DE 102 22 271 A1 Schichtdicken der Beschichtungsschicht von 10 µm bis 200 µm, US 6,029,344 Schichtdicken der Beschichtungsschicht zwischen ca. 6 µm und 125 µm.

Als besonders bevorzugt im Rahmen der vorliegenden Erfindung haben sich dagegen sehr dünne Hartstoffschichten aus metallischen Nitriden herausgestellt. Besonders bevorzugt sind hierbei Schichten aus Titannitrid, Titanaluminiumnitrid, Chromnitrid oder Zirconiumnitrid, ganz besonders bevorzugt Schichten aus Titanaluminiumnitrid oder Chromnitrid. Vorzugsweise weisen diese Schichten eine Schichtdicke von weniger als 2 µm, bevorzugt weniger als 1 µm, besonders bevorzugt weniger als 500 nm aus.

Überraschenderweise hat sich gezeigt, dass die vorteilhaften Wirkungen einer elektrisch leitenden Hartstoffoberfläche als Bestandteil einer elektrischen Kontaktverbindung, insbesondere in Bezug auf eine hohe und reproduzierbare Kontaktsicherheit auch nach vielen durchgeführten Kontaktierungsvorgängen, noch weiter verbessert werden können, wenn die Beschaffenheit der zweiten Kontaktfläche an die Beschaffenheit der Hartstoffoberfläche der ersten Kontaktfläche angepasst wird.

Es zeigte sich, dass bei Kontaktverbindungen, bei welchen beide Kontaktflächen Edelmetalloberflächen aufweisen, vor allem nach mehrmaligen Steckungen der Übergangswiderstand zwischen Testelement und Auswertegerät stark ansteigt, beispielsweise durch Materialablagerungen auf den Kontaktflächen, oder sogar kein elektrischer Kontakt mehr hergestellt werden kann. Solche Edelmetall-Edelmetall-Kontaktverbindungen werden weit verbreitet in bisherigen Testelement-Analysesystemen eingesetzt. Der Einsatz von Kontaktverbindungen mit Edelmetall-Edelmetall-Kontaktflächen in Testelement-Analysegeräten ist somit nur bedingt geeignet, insbesondere in Bezug auf eine hohe Genauigkeit und Reproduzierbarkeit der Analytbestimmung.

Durch den Einsatz einer Hartstoffoberfläche als Kontaktfläche in einer elektrischen Kontaktverbindung können die Nachteile solcher Kontaktverbindungen mit Edelmetall-Edelmetall-Kontaktflächen vermieden werden.

Als besonders vorteilhaft in Bezug auf eine hohe und reproduzierbare Kontaktsicherheit, insbesondere nach vielen Kontaktierungsvorgängen, hat sich überraschenderweise gezeigt, dass die Kontaktsicherheit dadurch weiter verbessert werden kann, dass nur eine Kontaktfläche mit einer Hartstoffoberfläche versehen ist und die zweite Kontaktfläche aus einem anderen Material besteht.

Insbesondere hat es ich als besonders vorteilhaft erwiesen, wenn die der mit einer Hartstoffoberfläche versehenen Kontaktfläche gegenüberliegende Kontaktfläche aus einem Material besteht, welches eine geringere Härte als das Material der Hartstoffoberfläche der anderen Kontaktfläche aufweist. Vorzugsweise eignen sich hierfür Metalle, insbesondere Edelmetalle wie Gold, Palladium oder Platin. Solche Materialien werden bereits weit verbreitet für Kontaktflächen, insbesondere Elektroden und Leiterbahnen, auf Testelementen eingesetzt. Dadurch ist es in vielen Fällen ausreichend, das Auswertegerät mit Kontaktelementen mit erfindungsgemäßen Hartstoffoberflächen bereitzustellen, in welches dann solche herkömmlichen Testelemente eingebracht werden können. Durch die Kombination einer Kontaktfläche mit einer Hartstoffoberfläche und einer Kontaktfläche aus einem Material, welches eine geringere Härte als das Material der Hartstoffoberfläche aufweist, kann eine hohe Reproduzierbarkeit des Übergangswiderstandes zwischen Testelement und Auswertegerät erreicht werden. Überraschend konnte in Dauerversuchen mit mehreren hunderten oder sogar tausenden Steckungen eines jeweils neuen Testelements mit Kontaktflächen aus Gold in ein erfindungsgemäße Kontaktelemente mit Chromnitrid-beschichteten oder Titanaluminiumnitrid-beschichteten Kontaktflächen beobachtet werden, dass die Übergangswiderstände jeweils stabil und auch nach vielen Steckungen unter 50 Ohm liegen. Bei mikroskopischer Betrachtung der Oberfläche des Testelements zeigt sich, dass, im Gegensatz zu Kontaktverbindungen mit Edelmetall-Edelmetall-Kontaktflächen, zu beobachten ist, dass die Kontaktfläche des Testelements zwar plastisch verformt wird, aber keine größeren Materialabtragungen oder Veränderungen der Schichtdicken zu beobachten sind. Insbesondere bleibt die Metallschicht der Elektroden und Leiterbahnen geschlossen erhalten. Eine möglichst geringe Verletzung der Kontaktfläche ist vor allem bei sehr dünnen Schichtdicken solcher Elektroden, Leiterbahnen oder Kontaktflächen auf Testelementen für eine exakte und reproduzierbare Analytbestimmung von entscheidender Bedeutung. Solche Testelemente sind beispielsweise elektrochemische Teststreifen, welche sehr dünne Metallschichten, beispielsweise Edelmetallkontakte mit Schichtdicken im Nanometerbis Mikrometerbereich auf einer elektrisch isolierenden Kunststofffolie aufweisen. Solche Metallschichten können beispielsweise mittels lithographischer Verfahren (Schichtdicken typischerweise 10 - 100 µm) oder Laserablation (Schichtdicken typischerweise 10 - 100 nm) auf solchen Unterlagen erzeugt werden. Bei solchen sehr dünnen elektrisch leitenden Metallschichten auf isolierenden und elastischen Oberflächen kann bereits eine geringe Abtragung dieser Schicht eine deutliche Erhöhung des Übergangswiderstandes oder im Extremfall eine vollständige Unterbrechung des elektrischen Kontakts bewirken. Mit den erfindungsgemäßen Hartstoffoberflächen der gegenüberliegenden Kontaktflächen werden deutlich geringere Schädigungen solcher Metallschichten erzielt, wodurch beim Einsatz in Testelement-Analysegeräten exakte und reproduzierbare Analytbestimmungen möglich sind.

Testelement-Analysesysteme werden vorzugsweise in analytischen und medizinischen Labors eingesetzt. Die Erfindung richtet sich jedoch auch auf Einsatzgebiete, bei denen die Analyse durch den Patienten selbst durchgeführt wird, um seinen Gesundheitszustand laufend zu überwachen ("home- monitoring"). Von besonderer medizinischer Bedeutung ist dies beispielsweise bei der Überwachung von Diabetikern, welche die Konzentration von Glucose im Blut mehrfach täglich bestimmen müssen oder von Patienten, welche gerinnungshemmende Medikamente einnehmen und deshalb ihren Koagulationsstatus in regelmäßigen Abständen bestimmen müssen. Für derartige Zwecke sollen die Auswertegeräte möglichst leicht und klein, batteriebetrieben und robust sein. Solche Testelement-Analysesysteme sind beispielsweise in DE 43 05 058 beschrieben.

Testelemente sind häufig als Teststreifen ausgebildet, welche im wesentlichen aus einer länglichen Trägerschicht, meist aus einem Kunststoffmaterial, und einer Messzone mit einer die Nachweisreagenzien enthaltenen Nachweisschicht und evtl. weiteren Hilfsschichten, beispielsweise Filtrationsschichten, bestehen. Weiterhin können Testelemente weitere Strukturelemente enthalten, beispielsweise Dosier- und Transportvorrichtungen für die Probe wie Kanäle oder Vliese, Positioniervorrichtungen wie Ausstanzungen zur Gewährleistung einer exakten Positionierung des Testelements und damit einer exakten Messung im Auswertegerät oder Kodierungselemente beispielsweise in Form eines Strichcodes oder eines elektronischen Bauteils, welche dazu dienen, spezifische Parameter des Testelements wie beispielsweise Kalibrationsdaten oder Chargeninformationen an das Auswertegerät zu übermitteln.

Testelemente enthalten zumeist in der Messzone Reagenzien, deren Reaktion mit der Probe, insbesondere mit dem in der Probe enthaltenen Analyten, zu einer charakteristischen und messbaren Veränderung des Testelements führt, welche mit dem zum System gehörigen Auswertegerät bestimmt werden kann. Die Messzone kann gegebenenfalls weitere Hilfsstoffe enthalten. Die Messzone kann auch nur Teile der Reagenzien oder Hilfsstoffe enthalten. In weiteren Fällen ist es möglich, dass die Nachweisreaktionen zur Bestimmung des Analyten nicht direkt in der Messzone ablaufen, sondern dass das Reagenzgemisch erst nach Ablauf der Nachweisreaktionen zur Messung in die Messzone gebracht wird. Dem mit der Technik von analytischen Testelementen oder diagnostischen Testträgern vertrauten Fachmann sind geeignete Reagenzien und Hilfsmittel zur Durchführung der analytspezifischen Nachweisreaktionen bestens bekannt. Für Analyten, die enzymatisch nachzuweisen sind, können beispielsweise Enzyme, Enzymsubstrate, Indikatoren; Puffersalze, inerte Füllstoffe und dergleichen mehr in der Messzone enthalten sein. Neben Nachweisreaktionen, die zu Farbveränderungen führen, sind dem Fachmann auch andere Nachweisprinzipien bekannt, die mit dem beschriebenen Testelement realisiert werden können, beispielsweise elektrochemische Sensoren oder chemische, biochemische, molekularbiologische, immunologische, physikalische, fluorimetrische oder spektroskopische Nachweismethoden. Der Gegenstand der vorliegenden Erfindung kann in allen diesen Nachweisverfahren eingesetzt werden. Dies gilt insbesondere für elektrochemische Analyseverfahren, bei welchen infolge einer analytspezifischen Nachweisreaktion eine elektrochemische, meist als Spannung oder Stromfluss messbare Veränderung in oder Messzone stattfindet.

Der Gegenstand der vorliegenden Erfindung kann, neben in solchen mit Reagenzien arbeitenden Analysesystemen, auch in reagenzfreien Analysesystemen eingesetzt werden, bei welchen nach Kontaktierung des Testelements mit der Probe eine charakteristische Eigenschaft der Probe (beispielsweise deren Ionenzusammensetzung mittels ionenselektiver Elektroden) ohne weitere Reagenzien direkt gemessen wird. Die Erfindung ist grundsätzlich auch für solche Analysesysteme verwendbar.

Weiterhin enthalten die Testelemente der vorliegenden Erfindung Kontaktflächen, welche elektrisch leitend sind und über welche ein elektrischer Kontakt zwischen dem Testelement und dem Auswertegerät hergestellt werden kann. Im Falle elektrochemischer Analyseverfahren sind insbesondere Leiterbahnen und Elektroden auf die Testelemente aufgebracht, mit deren Hilfe die elektrochemischen Veränderungen der Probe bestimmt sowie externe Spannungen und/oder Ströme an die zu untersuchende Probe angelegt werden können. Die elektrochemischen Analysen auf dem Testelement laufen insbesondere in der Messzone zwischen speziellen ausgebildeten Elektroden ab, während die von diesen ausgehenden elektrischen Messsignale beziehungsweise die an diese gerichteten Ansteuersignale über die Leiterbahnen gemessen beziehungsweise angelegt werden. Vorzugsweise enthalten diese Leiterbahnen speziell ausgestaltete flächige Bereiche, welche Kontaktflächen ausbilden, über welche der elektrische Kontakt zwischen Testelement und dem Auswertegerät hergestellt werden kann. Die Leiterbahnen und Kontaktflächen bestehen zumeist aus Edelmetallen. Auch Testelemente, welche keine elektrochemischen Analysemethoden einsetzen, können elektrisch leitende Kontaktflächen besitzen. Beispielsweise kann es vorteilhaft sein, auf einem Testelement elektronische Bauteile aufzubringen, welche dazu dienen, spezifische Parameter des Testelements wie beispielsweise Kalibrationsdaten oder Chargeninformationen zu speichern und an das Auswertegerät zu übermitteln. Hierzu werden diese spezifischen Daten auf dem Testelement in elektronischen Bauteilen oder Schaltkreisen gespeichert. Beim Einbringen des Testelements in das Auswertegerät können diese Daten von einer speziellen Leseelektronik des Auswertegerätes eingelesen und verarbeitet werden. Hierzu ist allerdings eine elektrische Kontaktierung des Testelements nötig, wozu wiederum die zuvor erwähnten elektrisch leitenden Kontaktflächen des Testelements unumgänglich sind.

Das Auswertegerät enthält eine Testelementhalterung, um ein Testelement in einer Messposition zur Durchführung der Messung zu positionieren. Diese Testelementhalterung enthält weiterhin die zuvor beschriebenen Kontaktelemente mit speziellen Kontaktflächen. Zur Bestimmung des Analyten wird das Testelement in ein Auswertegerät eingebracht, welches die durch den Analyten hervorgerufene charakteristische Veränderung des Testelements bestimmt und in Form eines Messwertes zur Anzeige oder Weiterverarbeitung bereitstellt. Die Bestimmung des Analyten kann mit verschiedensten, dem Fachmann auf dem Gebiet der apparativen Analytik bekannten Nachweismethoden durchgeführt werden. Insbesondere können optische und elektrochemische Nachweismethoden eingesetzt werden. Optische Methoden umfassen beispielsweise die Bestimmung charakteristischer Veränderungen der Messzone durch Messung von Absorption, Transmission, Circulardichroismus, optische Rotationsdispersion, Refraktrometrie oder Fluoreszenz. Elektrochemische Methoden können insbesondere auf der Bestimmung charakteristischer Ladungs-, Potential- oder Stromveränderungen in der Messzone beruhen.

Analyte, die mit einem erfindungsgemäßen Verfahren beziehungsweise den entsprechenden Vorrichtungen bestimmt werden können, sind im Sinne der vorliegenden Anmeldung alle Teilchen, die in der Analytik von Interesse sind, insbesondere in der klinischen Diagnostik. Insbesondere umfasst der Begriff "Analyte" Atome, Ionen, Moleküle und Makromoleküle, insbesondere biologische Makromoleküle wie Nukleinsäuren, Peptide und Proteine, Lipide, Metabolite, Zellen und Zellfragmente.

Unter der zur analytischen Untersuchung eingesetzten Probe versteht man im Sinne der vorliegenden Anmeldung sowohl ein unverändertes, den Analyten enthaltendes Medium als auch ein bereits verändertes, den Analyten oder davon abgeleitete Substanzen enthaltendes Medium. Die Veränderung des ursprünglichen Mediums kann insbesondere zum Aufschluss der Probe, zur Aufarbeitung des Analyten oder zur Durchführung der Nachweisreaktionen stattfinden, Bevorzugte Proben sind Flüssigkeiten. Flüssigkeiten können reine Flüssigkeiten und homogene oder heterogene Mischungen wie beispielsweise Dispersionen, Emulsionen oder Suspensionen sein. Insbesondere können in den Flüssigkeiten Atome, Ionen, Moleküle und Makromoleküle, insbesondere biologische Makromoleküle wie Nukleinsäuren, Peptide und Proteine, Lipide, Metabolite oder auch biologische Zellen oder Zellfragmente enthalten sein. Bevorzugte zu untersuchende Flüssigkeiten sind Körperflüssigkeiten wie Blut, Plasma, Serum, Urin, cerebrospinale Flüssigkeit, Tränenflüssigkeit, Zellensuspensionen, Zellüberstände, Zellextrakte, Gewebeaufschlüsse oder ähnliches. Flüssigkeiten können aber auch Kalibrationslösungen, Referenzlösungen, Reagenzlösungen oder Lösungen mit standardisierten Analytkonzentrationen, so genannte Standards, sein.

Unter analytischer Untersuchung oder Bestimmung von Analyten versteht man in der vorliegenden Anmeldung sowohl einen qualitativen als auch einen quantitativen Nachweis des Analyten. Insbesondere versteht man darunter eine Konzentrations- oder Mengenbestimmung der jeweiligen Analyten, wobei auch die alleinige Feststellung des Fehlens oder des Vorhandenseins des Analyten als analytische Untersuchung angesehen wird.

Die Erfindung wird nachfolgend anhand von Figuren und Ausführungsbeispielen näher erläutert. Die beschriebenen Eigenschaften und Besonderheiten können einzeln oder in Kombination verwendet werden, um bevorzugte Ausgestaltungen der Erfindung zu schaffen.
Figur 1 zeigt eine Teil-Schnittdarstellung eines erfindungsgemäßen Testelement-Analysesystems.
Figur 2 zeigt eine exemplarische Ansicht eines Testelements für elektrochemische Analyseverfahren.
Figur 3 zeigt eine Detailansicht eines erfindungsgemäßen Kontaktelements.
Figur 4 zeigt eine Detailansicht eines Querschnitts eines hartstoffbeschichteten Kontaktelements im Bereich der Kontaktfläche.
Figur 5 zeigt Häufigkeitsverteilungen experimentell bestimmter Übergangswiderstände zwischen Kontaktelementen mit nicht hartstoffbeschichteten Kontaktflächen aus elektropoliertem Palladium (Fig. 5a), zwischen Kontaktelementen mit Chromnitrid-beschichteten Kontaktflächen (Fig. 5b) oder Kontaktelementen mit Titanaluminiumnitrid-beschichteten Kontaktflächen (Fig. 5c) und jeweils neuen Testelementen mit Kontaktflächen aus 50 nm Gold.

Die Ziffern in den Figuren bedeuten:
- 1: Analysesystem
- 2: Auswertegerät
- 3: Testelement
- 4: Elektroden
- 5: Testelementhalterung
- 6: Federelement
- 7: Messzone
- 8: Flüssigkeitstropfen der Probe
- 9: Probenauftragszone
- 10: Transportzone
- 11: Kontaktfläche des Testelements
- 12: Kontaktfläche des Kontaktelements
- 13: Leiterbahn
- 14: Kontaktelement
- 15: Mess- und Auswerteelektronik
- 16: Leiterplatine
- 17: Spezial-IC
- 18: Grundmaterial
- 19: Zwischenschicht
- 20: Hartstoffschicht
- 21: Reagenzschicht

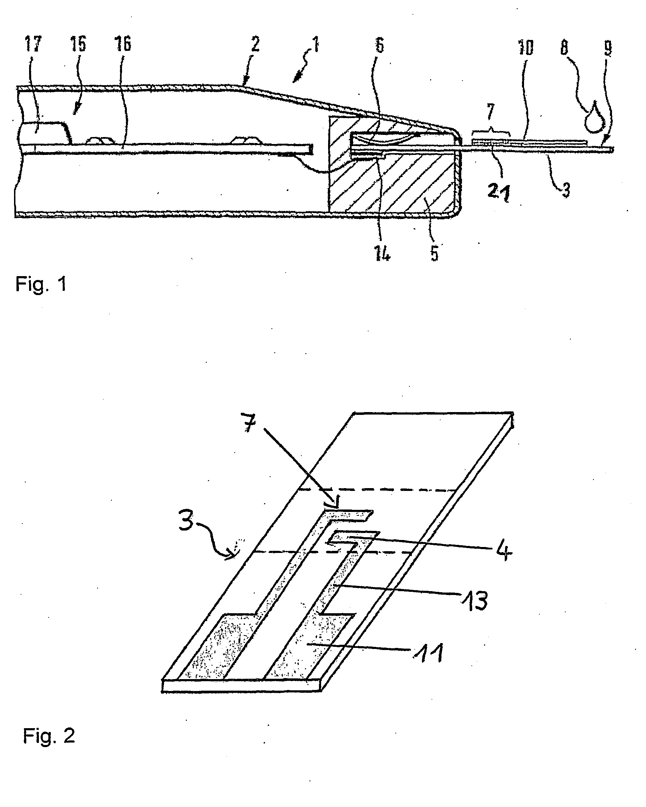

Das in Figur 1 dargestellte Testelement-Analysesystem 1 besteht aus einem Auswertegerät 2 und einem Testelement 3.

Das Auswertegerät 2 hat eine Testelementhalterung 5, mit welcher ein Testelement 3 in der in Figur 1 dargestellten Messposition positioniert wird. Das Testelement 3 ist durch geeignete Mittel, beispielsweise durch ein Federelement 6, in der Messposition fixiert.

Zur Durchführung einer Messung wird Probenflüssigkeit in die Messzone 7 des Testelements 3 gebracht. Bei der dargestellten Ausführungsform geschieht dies dadurch, dass ein Flüssigkeitstropfen 8 auf eine am Ende des Testelements 3 vorgesehene Probenauftragszone 9 aufgebracht und von dort durch eine Transportzone 10, beispielsweise einen Kapillarspalt, zu der Messzone 7 transportiert wird. In der Messzone 7 befindet sich eine Reagenzschicht 21, die von der Probenflüssigkeit aufgelöst wird und mit deren Bestandteilen reagiert.

Die Reaktion führt zu einer detektierbaren Veränderung in der Messzone 7. Im Fall eines elektrochemischen Testelements erfolgt die Bestimmung der elektrischen Messgrösse mittels in der Messzone vorgesehener, in Figur 2 dargestellter Elektroden. In der Messposition wird ein elektrischer Kontakt zwischen dem Testelement 3 und dem Kontaktelement 14 der Testelementelementhalterung 5 hergestellt. Das Kontaktelement 14 ist mit einer Mess- und Auswerteelektronik 15 verbunden, die im Hinblick auf eine möglichst kompakte Bauweise und hoher Zuverlässigkeit hochintegriert ist Im dargestellten Fall besteht sie im Wesentlichen aus einer Leiterplatine 16 und einem Spezial-IC 17. Insoweit ist das Analysesystem konventionell konstruiert und bedarf keiner näheren Erläuterung.

Figur 2 zeigt eine Teilansicht eines exemplarischen Testelements 3 für elektrochemische Analyseverfahren.

Der Nachweis einer analytspezifischen Veränderung erfolgt im Rahmen der Analytbestimmung innerhalb der Messzone 7. Im dargestellten Fall eines elektrochemischen Testelements erfolgt die Messung einer elektrischen Messgrösse mittels in der Messzone vorgesehener Elektroden 4. Das elektrische Signal wird über die Leiterbahnen 13 an die Kontaktflächen 11 weitergeleitet. Diese treten,beim Einstecken des Testelements 3 in die Testelementhalterung 5 mit den Kontaktflächen des Kontaktelements 12 in direkten Kontakt und stellen so die elektrische Verbindung zwischen Testelement und Auswertegerät her. Das hier dargestellte Testelement stellt lediglich eine beispielhafte und minimalisierte Ausführungsform eines Teststreifens dar. Auch Testelemente mit anderen Elektroden- und Leiterbahnenanordnungen sowie mehreren Elektroden, beispielsweise Referenzelektroden, sowie zusätzlichen Strukturen, wie Probenauftrags- oder Transportzonen oder speziellen Reaktionsbereichen, können im Rahmen der vorliegenden Erfindung eingesetzt werden.

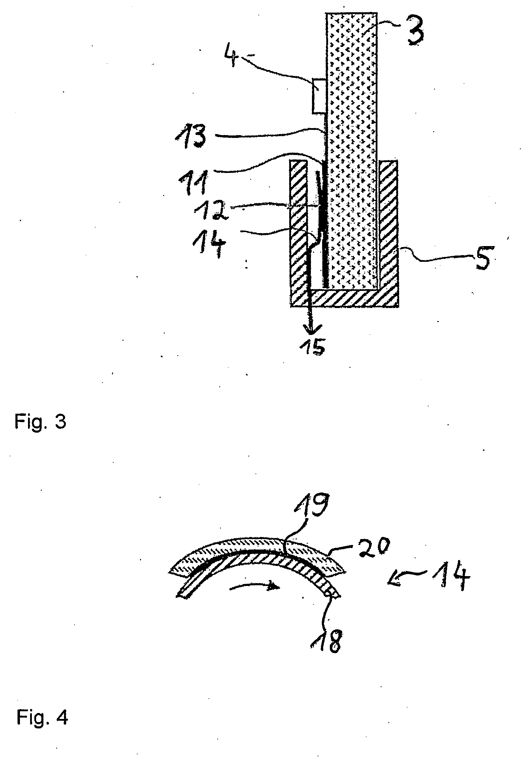

Figur 3 zeigt eine Detailansicht eines erfindungsgemäßen Kontaktelements.

Das Testelement 3 wird durch Einschieben in die Testelementhalterung 5 eingebracht. Der elektrische Kontakt wird zwischen den Kontaktflächen des Kontaktelements 12 und den Kontaktflächen des Testelements 11 hergestellt. Hierbei ist insbesondere das Kontaktelement 14 derartig ausgeformt, dass es federnde Eigenschaften besitzt und so einen definierten Kontaktdruck auf das Testelement 3 ausübt. Dies zeigt eine besonders bevorzugte Ausführungsform, in welcher das Kontaktelement 14 sowohl für die elektrische Kontaktierung als auch für eine Positionierung und Fixierung des Testelements sorgt. Dem gegenüber sind in Figur 1 die Funktionen elektrische Kontaktierung und Positionierung/Fixierung auf zwei unterschiedliche Bauteile, nämlich das Federelement 6 und das Kontaktelement 14 verteilt.

Figur 4 zeigt eine Detailansicht eines Querschnitts eines hartstoffbeschichteten Kontaktelements im Bereich der Kontaktfläche. Die Hartstoffbeschichtung 20 ist hierbei auf das Grundmaterial 18 des Kontaktelements aufgebracht, wobei im vorliegenden Fall zwischen den beiden Schichten eine Zwischenschicht 19 vorliegt, welche insbesondere als Haftvermittler- oder Schutzschicht ausgebildet sein kann. Die Hartstoffbeschichtung 20 entspricht funktionell der Kontaktfläche des Kontaktelements 12.

### Beispiele:

Für einen Einsatz in Testelement-Analysesystemen ist es wichtig, dass die Kontaktverbindungen zwischen Testelementen und Analysegerät auch nach vielfachen Steckungen noch definierte und geringe Übergangswiderstände garantieren, um so eine exakte und reproduzierbare Analytbestimmung mittels des Testelement-Analysegeräts zu gewährleisten.

### Beispiel 1: Kontaktelementen mit Chromnitrid-beschichteten Kontaktflächen

### a) mikroskopische Oberflächenuntersuchung

Um die vorteilhafte Wirkung erfindungsgemäßer Hartstoffoberflächen als Kontaktflächen in einer elektrischen Kontaktverbindung eines Testelement-Analysesystems zu belegen, wurden die Kontaktflächen solcher Steckverbindungen mit Chromnitrid beschichtet. Hierbei wurde auf die Kontaktflächen der Steckverbindung mittels PVD-Verfahrens eine 480 nm dicke Chromnitridschicht aufgebracht. In diese derartig beschichteten Steckverbindungen wurden 480x jeweils neue Testelemente eingesteckt. Diese Testelemente besaßen als Kontaktflächen Goldschichten einer Dicke von 50 nm, welche auf eine Kunststofffolie aufgebracht waren. Nach den 480 Steckvorgängen wurde sowohl das Schädigungsbild der Kontaktflächen der Steckverbindung als auch der einzelnen Kontaktflächen der Testelemente mikroskopisch ausgewertet. Zum Vergleich wurden 480 Testelemente unter sonst identischen Bedingungen in herkömmliche, nicht hartstoffbeschichtete Steckverbindungen mit Kontaktflächen aus elektropoliertem Palladium gesteckt und ebenfalls eine mikroskopische Auswertung vorgenommen.

Das Schädigungsbild der Kontaktflächen der Testelemente, welche in herkömmliche Steckverbindungen mit Kontaktflächen aus elektropoliertem Palladium gesteckt wurden, zeigte sehr starke Materialabtragungen und Verformungen der Goldschicht der Testelemente. Diese entstanden durch das zum Testelement relative Darüberhinwegbewegen der Steckverbindung. So wird in vielen Bereichen die Goldschicht der Kontaktflächen und Leiterbahnen des Testelements durch die sich relativ darüber hinwegbewegende zweite Kontaktfläche der Steckverbindung stark verformt. Diese Verformung kann soweit gehen, dass in manchen Bereichen die Goldschicht durch das Darüberhinwegkratzen der zweiten Kontaktfläche bis zur Kunststofffolie abgetragen wird. Dadurch ist in diesen Fällen der elektrische Kontakt unterbrochen und eine Analytbestimmung damit unmöglich.

Das Schädigungsbild der Kontaktflächen der Testelemente, welche in erfindungsgemäße Steckverbindungen, deren Kontaktflächen mit 480 nm Chromnitrid beschichtet waren, gesteckt wurden, zeigte hingegen deutlich geringere Schädigungen. Bei mikroskopischer Betrachtung der Kontaktflächen des Testelements zeigte sich, dass die Kontaktflächen des Testelements zwar plastisch verformt waren, aber in weit geringerem Maße als bei den nicht hartstoffbeschichteten Steckverbindungen. Insbesondere waren keine größeren Materialabtragungen oder Veränderungen der Schichtdicken zu beobachten und die Goldschichtschicht der Kontaktfläche und Leiterbahnen blieb in allen Fällen geschlossen erhalten. Das mikroskopische Schädigungsbild zeigte eine gleichmäßigere Verformung der Goldschicht in Form einer flachen Rinne mit relativ gleich bleibender und geringer Tiefe, ohne dass das Material an einigen Stellen stark oder gar komplett abgetragen wäre.

Das mikroskopische Schädigungsbild herkömmlicher, nicht hartstoffbeschichteter Kontaktflächen der Kontaktelemente der Steckverbindungen nach 480 Steckungen war ebenfalls deutlich schlechter als das mikroskopische Schädigungsbild entsprechender Kontaktflächen mit erfindungsgemäßer Hartstoffoberfläche aus 480 nm Chromnitrid.

Die nicht hartstoffbeschichteten Kontaktflächen der Kontaktelemente der KontrollSteckverbindungen zeigten bei mikroskopischer Betrachtung deutliche Abrieberscheinungen der Metallschicht, welche bis zum stellenweise vollständigen Abrieb der Metallschicht führten. Teilweise waren auch Ablagerungen von Oberflächenmaterialen der eingesteckten Testelemente auf den Kontaktflächen der Steckverbindungen zu beobachten.

Die Chromnitrid-beschichteten Kontaktflächen der erfindungsgemäßen Kontaktelemente zeigten dagegen auch nach derartig häufigen Steckungen keine deutlichen Verschleißerscheinungen. Insbesondere war eine intakte und durchgehende Oberfläche der Chromnitridschicht sowie eine verminderte Ablagerung des Elektrodenmaterials des Teststreifens zu beobachten.

### b) elektrische Übergangswiderstände

Bei der Messung elektrischer Übergangswiderstände zwischen Testelement und Kontaktelement der Steckverbindung zeigt sich insbesondere nach vielen Steckungen, dass hartstoffbeschichtete Kontaktelemente deutlich reproduzierbare und weniger störanfällige elektrische Kontaktverbindungen ermöglichen als herkömmliche Kontaktelemente ohne eine solche Hartstoffoberfläche. Hierzu wurden jeweils 480 neue, oben beschriebene Testelemente, welche eine 50 nm dicke Goldschicht als Elektrodenmaterial besaßen, entweder in Steckverbindungen, deren Kontaktflächen mit 480 nm Chromnitrid beschichtet waren oder in Steckverbindungen mit nicht hartstoffbeschichteten Kontaktflächen aus elektropoliertem Palladium (Kontrollen) gesteckt und jeweils die Übergangswiderstände zwischen 8 Elektrodenflächen eines Testelements und der Steckverbindung bestimmt, so dass je 3840 Werte der Übergangswiderstände für hartstoffbeschichtete Kontaktflächen und nicht hartstoffbeschichtete Kontroll-Kontaktflächen ermittelt wurden.

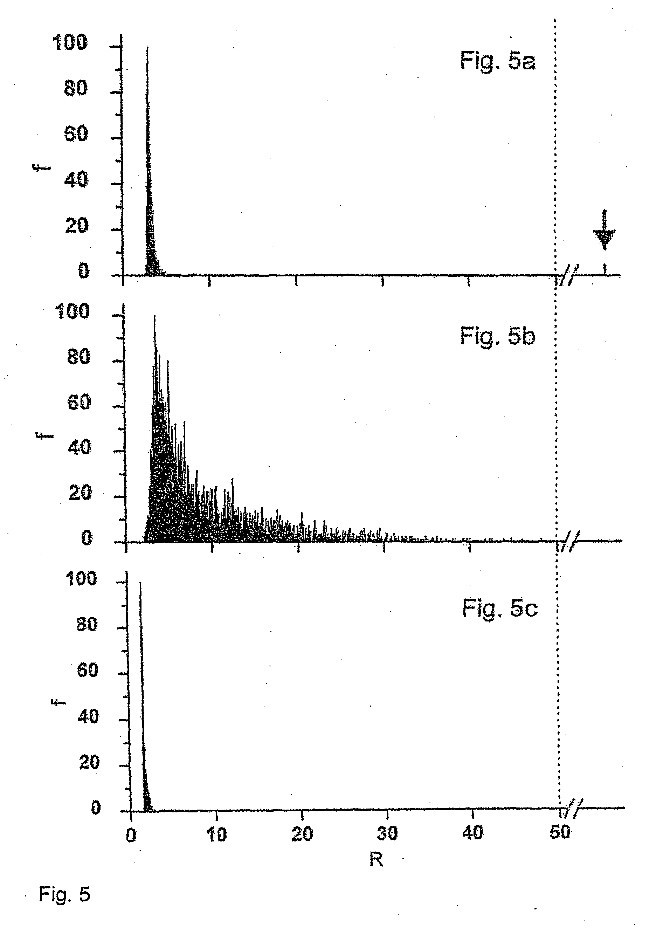

Figur 5a zeigt eine Häufigkeitsverteilung der so bestimmten Übergangswiderstände für Kontaktelemente mit nicht hartstoffbeschichteten Kontaktflächen aus elektropoliertem Palladium. Auf der Abszisse ist der jeweilige gemessene Übergangswiderstand R in 0,1 Ohm-Bereichen und auf der Ordinate die auf den größten Häufigkeitswert (=100) normalisierte Häufigkeit f der gemessen Übergangswiderstände aufgetragen.

Es zeigt sich, dass sich der Großteil der Übergangswiderstände bei diesen Kontrollen zwar im Bereich geringer Werte zwischen 0 und 10 Ohm befindet, aber auch eine nicht unerhebliche Anzahl gemessener Übergangswiderstände eine "unendliche" Größe erreicht. Solche als "unendlich" gemessenen Übergangswiderstände bedeuten, dass kein elektrischer Kontakt zwischen dem jeweiligen Kontaktelement der Steckverbindung und der Kontaktfläche des Testelements besteht und keine Widerstandsmessung durchgeführt werden kann. Diese Werte befinden sich in Fig. 5a auf der rechten Seite und sind durch den Pfeil markiert. Die Auswertung der einzelnen gemessenen Übergangswiderstände ergab, dass 32 der gemessenen Übergangswiderstände größer als 50 Ohm waren. Ein Übergangswert von 50 Ohm kann in derartig aufgebauten Testelement-Analysesystemen als Grenzwert angesehen, unterhalb dessen eine Messung noch als zu zuverlässig angesehen werden kann. 16 der untersuchten 480 Testelemente wiesen jedoch mindestens einen Übergangswiderstandswert über 50 Ohm auf. Dies resultiert in einer hohen Fehlerquote von 3,3% der gemessenen Testelemente, so dass derartige Kontaktelemente nur bedingt für einen dauerhaften und reproduzierbaren Einsatz in Testelement-Analysesystemen geeignet sind.

Figur 5b zeigt eine Häufigkeitsverteilung der gemessenen Übergangswiderstände für erfindungsgemäße Kontaktelemente, welche mit 480 nm Chromnitrid hartstoffbeschichtet waren.

Deutlich zeigt sich, dass bei der Bestimmung der 3840 Übergangswiderstände keine Werte größer als 50 Ohm zu beobachten waren. Zwar liegt mehr Widerstandswerte zwischen 10 und 50 Ohm, doch keiner der gemessenen 3840 Werte befindet sich oberhalb des Grenzwertes von 50 Ohm oder gar bei "unendlichen" Übergangswiderstandswerten wie bei den nicht hartstoffbeschichteten Kontaktflächen. Mit solchen Kontaktverbindungen kann somit eine reproduzierbare und exakte Übermittlung elektrischer Signale über viele Steckvorgänge hinweg erfolgen. Der Einsatz solcher erfindungsgemäßer Kontaktverbindungen in Testelement-Analysegeräten hat damit den Vorteil, dass in solchen Systemen reproduzierbare und exakte Analytbestimmungen durchgeführt werden können, insbesondere auch nach vielfachen erfolgten Steckungen.

### Beispiel 2: Kontaktelemente mit Titannitrid-beschichteten Kontaktflächen

Analoge Versuche wurden mit Steckverbindungen mit Kontaktelementen durchgeführt, deren Kontaktflächen eine erfindungsgemäße Hartstoffoberfläche aus 120 nm Titanaluminiumnitrid, welche ebenfalls mittels PVD-Verfahren aufgebracht wurde, aufweisen.

### a) mikroskopische Oberffächenuntersuchung

Auch hier zeigte sich, dass eine erfindungsgemäße Hartstoffbeschichtung der Kontaktflächen der Steckverbindung mit Titanaluminiumnitrid das Schädigungsbild der Kontaktflächen der Testelemente deutlich verbessert. Bei mikroskopischer Betrachtung der jeweiligen Kontaktflächen des Testelements zeigte sich, dass die Kontaktfläche des Testelements zwar plastisch verformt wurde, aber in weit geringerem Maße als bei den nicht hartstoffbeschichteten Kontrollen. Insbesondere waren keine größeren Materialabtragungen oder Veränderungen der Schichtdicken zu beobachten und die Goldschichtschicht der Kontaktflächen und Leiterbahnen blieb geschlossen erhalten. Das mikroskopische Schädigungsbild zeigte, ähnlich wie bei einer Chromnitrid-beschichteten Kontaktfläche, eine gleichmäßige Verformung der Goldschicht in Form einer flachen Rinne mit relativ gleich bleibender und geringer Tiefe, ohne dass das Material an einigen Stellen stark oder gar komplett abgetragen wäre.

Auch die Schädigungen an der Titanaluminiumnitridbeschichtung selbst waren nach mehr als 480 Steckungen deutlich geringer als bei den nicht hartstoffbeschichteten Kontrollsteckverbindungen. Die Titanaluminiumnitrid-beschichteten Kontaktflächen der erfindungsgemäßen Steckverbindungen zeigten auch nach häufigen Steckungen keine merkbaren Abrieberscheinungen oder Ablagerungen. Insbesondere war noch eine intakte und durchgehende Oberfläche der Titannitridschicht zu beobachten.

### b) elektrische Übergangswiderstände

Bei der Messung von Übergangswiderständen analog zur Messung aus Beispiel 1 b) zeigt sich, dass mit Titanaluminiumnitrid beschichtete Kontaktflächen von Steckverbindungen deutlich reproduzierbare und wesentlich weniger störanfällige elektrische Kontaktverbindungen zwischen Steckverbindung und Testelement ermöglichen als herkömmliche Steckverbindungen ohne eine solche Hartstoffoberfläche. Besonders vorteilhaft bei dieser Ausführungsform ist, dass neben einer sehr hohen Kontaktsicherheit (keine Werte oberhalb 50 Ohm) im Vergleich zu den Chromnitrid-beschichteten Kontaktflächen aus Beispiel 1 die Werte der gemessenen Übergangswiderstände noch deutlich geringer sind.

Figur 5c zeigt eine Häufigkeitsverteilung der gemessenen Übergangswiderstände für erfindungsgemäße Kontaktelemente mit mit 120 nm Titanaluminiumnitrid hartstoffbeschichteten Kontaktflächen.

Deutlich zeigt sich, dass bei der Bestimmung der Übergangswiderstände keine Werte größer als 50 Ohm zu beobachten waren. Auch liegen fast alle gemessenen Widerstandswerte zwischen 1 und 3 Ohm und somit sogar noch deutlich geringer als bei den nicht hartstoffbeschichteten Kontaktflächen aus Beispiel 1 (Fig. 5a) Mit solchen Kontaktverbindungen kann somit eine reproduzierbare und exakte Übermittlung elektrischer Signale über viele Steckvorgänge hinweg erfolgen. Der Einsatz solcher erfindungsgemäßer Kontaktverbindungen in Testelement-Analysegeräten hat damit den Vorteil, dass in solchen Systemen hiermit reproduzierbare und exakte Analytbestimmungen durchgeführt werden können, insbesondere auch nach vielfachen erfolgten Steckungen. Die sehr geringen Übergangswiderstände bei Kontaktelementen mit Titanaluminiumnitrid-beschichteten Kontaktflächen haben weiterhin den Vorteil, dass das eigentliche Messsignal kaum durch den Übergangswiderstand beeinflusst wird und so besonders exakte Analytbestimmungen auch mit niedrigen Messsignalen durchgeführt werden können.

### Beispiel 3: Änderung der Übergangswiderstände aus Beispiel 1 und 2 nach vielfachen Steckungsvorgängen:

Für einen reproduzierbaren Einsatz in Testelement-Analysesystemen über einen langen Zeitraum und viele Messzyklen hinweg ist es weiterhin wichtig, dass die Übergangswiderstände möglichst konstant sind und kein extremes Einlauf- oder Verschleißverhalten zeigen, welches mit zunächst hohen und dann sinkenden Widerstandswerten oder zunächst geringen und dann steigenden Widerstandswerten gekennzeichnet ist.

Kontrollsteckverbindungen mit Kontaktflächen aus elektropoliertem Palladium zeigten über die 480 Steckungen eines jeweils neuen Testelements über den gesamten Zeitbereich verstreut Kontaktausfälle, welche durch "unendliche" Widerstandswerte gekennzeichnet sind. Solche zufällig verstreuten Kontaktausfälle sind insbesondere für die Reproduzierbarkeit der Messergebnisse ungünstig, da immer mit einer gewissen Wahrscheinlichkeit mit einem Messfehler gerechnet werden muss.

Steckverbindungen mit Kontaktflächen, welche mit 480 nm Chromnitrid beschichtet waren, zeigen eine Zunahme der Übergangswiderstände mit zunehmender Anzahl erfolgter Steckungen. Bei derartigen Testsystemen kann es daher vorteilhaft sein, die hartstoffbeschichteten Kontaktelemente nach einer bestimmten Anzahl von Messungen auszutauschen, um so eine gleich bleibende hohe Messgenauigkeit zu gewährleisten. Aus diesem Grunde sind auch Auswertegeräte, welche solche hartstoffbeschichteten Kontaktelemente und Kontaktverbindungen enthalten, in der vorliegenden Erfindung enthalten.

Steckverbindungen mit Kontaktflächen, welche mit 120 nm Titanaluminiumnitrid beschichtet waren, zeigen zunächst höhere Übergangswiderstände, welche mit steigender Anzahl von Steckungen auf ein sehr niedriges Widerstandsniveau abfallen. Diese Beobachtung zeigt, dass durch eine Titanaluminiumnitridbeschichtung im Gegensatz zu einer Chromnitridbeschichtung vor allem noch nach vielen durchgeführten Steckungen sehr reproduzierbare und geringe Überstandswiderstände erzielt werden können und sich solche Beschichtungen der Kontaktflächen sich insbesondere für Testelement-Analysesysteme eignen, welche über eine sehr lange Nutzungsdauer und sehr viele Messungen hinweg exakte Analytbestimmungen ermöglichen sollen.

Dies zeigt, dass es durch die Wahl des Materials der Hartstoffoberfläche weiterhin möglich ist, das Testelement-Analysesystem der Anforderungen des jeweiligen Einsatzbereiches, insbesondere in Bezug auf Lebensdauer und Exaktheit der Analytbestimmung anzupassen.

## Patentansprüche

1. Testelement-Analysesystem (1) zur analytischen Untersuchung einer Probe, insbesondere einer Körperflüssigkeit, umfassend
- ein Testelement (3) mit mindestens einer Messzone (7) und elektrisch leitenden Kontaktflächen (11), insbesondere Elektroden oder Leiterbahnen, wobei die zu untersuchende Probe zur analytischen Untersuchung in die Messzone (7) gebracht wird, sowie
- ein Auswertegerät (2) mit einer Testelementhalterung (5) zur Positionierung des die Probe enthaltenden Testelements (3) und einer Messvorrichtung zur Messung einer für den Analyten charakteristischen Veränderungen in der Messzone, wobei die Testelementhalterung (5) Kontaktelemente (14) mit Kontaktfächen (12) enthält, welche einen elektrischen Kontakt zwischen dem Kontakttlächen (11) des Testelements (3) Kind den Kontaktflächen (12) der Testelementhalterung (5) vermöglichen,
**dadurch** gekenntzeichnet, dass
die Kontaktflächen (12) der Kontaktelemente (14) der Testelementhalterung (5) mit einer elektrisch leitenden Hartstoffoberfläche (20) wersehen sind.

2. Testelement-Analysesystem gemäß Anspruch 1,
**dadurch gekennzeichnet, dass**
die der Hartstoffoberfläche (20) gegenüberliegende Kontaktfläche (11) aus einem Material besteht, welches unterschiedlich zum Material der Hartstoffoberfläche (20) ist.

3. Testelement-Analysesystem gemäß einem der vorherigen Ansprüche,
**dadurch gekennzeichnet, dass**
die der Hartstoffobertläche (20) gegenüberliegende Kontaktfläche (11) aus einem Material, insbesondere aus einen Edelmetall oder einer edelanetallhaltigen Legierung, besteht, welches eine geringere Härte als das Material der Hartstoffoberfläche (20) aufweist.

4. Testelement-Analysesystem gemäß einem der vorherigen Ansprüche,
**dadurch gekennzeichnet, dass**
die Kontaktelemente (14) der Testelementhalterung (5) als Steckkontakte, Federkontakte oder Klemmkontakte ausgebildet sind.

5. Testelement-Analysesystem gemäß einem der vorherigen Ansprüche,
**dadurch gekennzeichnet, dass**
die Hartstoffoberfläche(20) aus einem metallischen Nitrid, insbesondere Titannitrid, Titanaluminiumnitrid, Chromnitrid oder Zirconiumnitrid, besteht.

6. Testelement-Analysesystem gemäß einem der vorherigen Ansprüche,
**dadurch gekennzeichnet dass**
die Hartstoffoberfläche (20) durch Beschichtung eines Grundmaterials (18) mit einem elektrisch leitenden Hartstoff erzeugt wird.

7. Testelement-Analysesystem gemäß Anspruch 6,
**dadurch gekennzeichnet, dass**
zwischen dem Grundmaterial (18) und der Hartstoffoberfläche (20) weitere Zwischenschichien (19), insbesondere Haftvermittler- oder Schutzschichten, vorhanden sind.

8. Testelement-Analysesystem gemäß einem Ansprüche 6 bis 7,
**dadurch gekennzeichnet, dass**
die Schichtdicke der Hartstoffbeschichtung weniger als 2 µm, bevorzugt weniger als 1 µm, besonders bevorzugt weniger als 500 nm beträgt.

9. Testelement-Analysesystem gemäß einem der vorherigen Ansprüche,
**dadurch gekennzeichnet,**
**dass** der elektrische Ubergangswiderstand zwischen den Kontaktflächen (11) des Testelements (3) und den Kontaktflächen (12) der Testelementhalterung (5) Weniger als 50 Ohm beträgt.

10. Testelement-Analysesystem gemäß einem der vorherigen Ansprüche,
**dadurch gekennzeichnet,**
**dass** die Bestimmung des Analyten mit elektrischen, insbesondere elektrochemischen Methoden erfolgt.

## Claims

1. Test element analysis system (1) for the analytical examination of a sample, in particular of a body fluid comprising
- a test element (3) with at least one measuring zone (7) and electrically conductive contact surfaces (11) in particular electrodes or conductive paths where the sample to be examined is brought into the measuring zone (7) for the analytical examination,
as well as
- an evaluation device (2) with a test element holder (5) for positioning the test element (3) containing the sample and a measuring device for measuring a change in the measuring zone that is characteristic for the analyte wherein the test element holder (5) contains contact elements (14) with contact surfaces (12) which allow an electrical contact between the contact surfaces (11) of the test element (3) and the contact surfaces (11) of the test element holder (5),
**characterized in that**
the contact surfaces (12) of the contact elements (14) of the test element holder (5) are provided with an electrically conductive hard material surface (20).

2. Test element analysis system according to claim 1,
**characterized in that**
the contact surface (11) opposite to the hard material surface (20) consists of a material which is different from the material of the hard material surface (20).

3. Test element analysis system according to one of the previous claims,
**characterized in that**
the contact surface (11) opposite to the hard material surface (20) consists of a material and in particular of a noble metal or an alloy containing a noble metal which is of lower hardness than the material of the hard material surface (20).

4. Test element analysis system according to one of the previous claims,
**characterized in that**
the contact elements (14) of the test element holder (5) are in the form of plug contacts, spring contacts or clamping contacts.

5. Test element analysis system according to one of the previous claims,
**characterized in that**
the hard material surface (20) consists of a metallic nitride, in particular titanium nitride, titanium aluminium nitride, chromium nitride or zirconium nitride.

6. Test element analysis system according to one of the previous claims,
**characterized in that**
the hard material surface (20) is produced by coating a base material (18) with an electrically conductive hard material.

7. Test element analysis system according to claim 6,
**characterized in that**
additional interlayers (19) in particular adhesive or potective layers are present between the base material (18) and the hard material surface (20).

8. Test element analysis system according to one of the claims 6 to 7,
**characterized in that**
the layer thickness of the hard material coating is less than 2 µm, preferably less than 1 µm, particularly preferably less than 500 nm.

9. Test element analysis system according to one of the previous claims,
**characterized in that**
the electrical transition resistance between the contact surfaces (11) of the test element (3) and the contact surfaces (12) of the test element holder (5) is less than 50 ohms.

10. Test element analysis system according to one of the previous claims,
**characterized in that**
the analyte is determined using electrical methods and in particular electrochemical methods.

## Revendications

1. Système d'analyse d'élément de test (1) pour l'examen analytique d'un échantillon, en particulier d'un liquide corporel, comprenant
- un élément de test (3) comprenant au moins une zone de mesure (7) et des surfaces de contact électroconductrices (11), en particulier des électrodes ou des pistes conductrices, l'échantillon à analyser étant amené dans la zone de mesure (7) pour l'examen analytique ; et
- un appareil d'évaluation (2) comprenant un dispositif de fixation de l'élément de test (5) pour le positionnement de l'élément de test (3) contenant l'échantillon et un dispositif de mesure pour la mesure d'une modification dans la zone de mesure, caractéristique pour l'analyte, le dispositif de fixation de l'élément de test (5) contenant des éléments de contact (14) comprenant des surfaces de contact (12) qui permettent d'établir un contact électrique entre les surfaces de contact (11) de l'élément de test (3) et les surfaces de contact (12) du dispositif de fixation de l'élément de test (5) ;
**caractérisé en ce que**
les surfaces de contact (12) des éléments de contact (14) du dispositif de fixation de l'élément de test (5) sont munies d'une surface en substance dure (20) électroconductrice.

2. Système d'analyse d'élément de test selon la revendication 1,
**caractérisé en ce que**
la surface de contact (11) opposée à la surface en substance dure (20) est constituée d'une matière qui est différente de la matière de la surface en substance dure (20).

3. Système d'analyse d'élément de test selon l'une quelconque des revendications précédentes, **caractérisé en ce que**
la surface de contact (11) opposée à la surface en substance dure (20) est constituée d'une matière, en particulier d'un métal noble ou d'un alliage de métaux nobles, qui possède une dureté différente de celle de la matière de la surface en substance dure (20).

4. Système d'analyse d'élément de test selon l'une quelconque des revendications précédentes, **caractérisé en ce que**
les éléments de contact (14) du dispositif de fixation de l'élément test (5) sont réalisés sous la forme de contacts à fiche, de contacts à ressort ou de contacts de borne.

5. Système d'analyse d'élément de test selon l'une quelconque des revendications précédentes, **caractérisé en ce que**
la surface en substance dure (20) est constituée par un nitrure métallique, en particulier le nitrure de titane, le nitrure de titane-aluminium, le nitrure de chrome ou le nitrure de zirconium.

6. Système d'analyse d'élément de test selon l'une quelconque des revendications précédentes, **caractérisé en ce que**
on obtient la surface en substance dure (20) par enduction d'une matière de base (18) avec une substance dure électroconductrice.

7. Système d'analyse d'élément de test selon la revendication 6, **caractérisé en ce que**
entre la matière de base (18) et la surface en substance dure (20), on prévoit d'autres couches intermédiaires (19), en particulier des couches promotrices d'adhérence ou des couches de protection.

8. Système d'analyse d'élément de test selon l'une quelconque des revendications 6 à 7, **caractérisé en ce que**
l'épaisseur de couche de l'enduction en substance dure est inférieure à 2 µm, de préférence inférieure à 1 µm, de manière particulièrement préférée inférieure à 500 nm.

9. Système d'analyse d'élément de test selon l'une quelconque des revendications précédentes, **caractérisé en ce que**
la perte de tension électrique au passage entre les surfaces de contact (11) de l'élément de test (3) et les surfaces de contact (12) du dispositif de fixation de l'élément de test (5) est inférieure à 50 ohm.

10. Système d'analyse d'élément de test selon l'une quelconque des revendications précédentes, **caractérisé en ce que**
la détermination de l'analyte a lieu avec des procédés électriques, en particulier électrochimiques.
